# EUROPEAN PATENT SPECIFICATION

(11) **EP 4 347 615 B1**
(45) Date of publication and mention of the grant of the patent: **21.05.2025**
(21) Application number: 22732086.8
(22) Date of filing: 01.06.2022
(51) Int. Cl.: C07K 5/06

(54) **PROCESS FOR MAKING MELFLUFEN OR A SALT THEREOF**
VERFAHREN ZUR HERSTELLUNG VON MELFLUFEN ODER EINEM SALZ DAVON
PROCÉDÉ DE PRÉPARATION DE MELFLUFÈNE OU D'UN SEL DE CELUI-CI

(30) Priority: 01.06.2021 EP 21177148
(43) Date of publication of application: 10.04.2024
(73) Proprietor: Synthon B.V., 6545 CM Nijmegen (NL)
(72) Inventor: BARTOS, Petr, 67817 Blansko (CZ); CASTULIK, Jakub, 67817 Blansko (CZ)
(74) Representative: Dobsík, Martin
(86) International application number: PCT/EP2022/064901
(87) International publication number: WO 2022/253894

(56) References cited:
- WO-A1-2016/180740
- WO-A1-2020/079165

## Description

### BACKGROUND OF THE INVENTION

The present invention relates to an improved process for preparing the compound Melflufen, chemically 4-[N,N-Bis(2-chloroethyl)amino]-L-phenylalanyl-4-fluoro-L-phenylalanine ethyl ester, compound of formula (1), or a salt thereof,

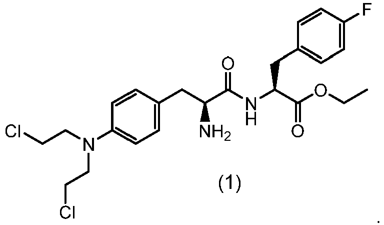

Melflufen, compound of formula (1) is a DNA alkylating drug. Melflufen is useful in the treatment of multiple myeloma. Melflufen is marketed in form of HCl salt under the brand name Pepaxto by Oncopeptides.

Melflufen was first disclosed in WO2001096367 by Oncopeptides. The application describes a process for preparation of Melflufen starting form L-Melphalan. L-Melphalan is highly toxic compound, use thereof is not suitable for high scale preparation. The process also comprises purification using column chromatography that is also not suitable for high scale preparation. Another process for preparation of Melflufen is described in WO2016180740 application by Oncopeptides. The applicant discloses that chlorination of compound of formula (2), 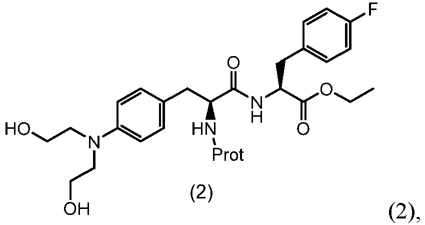 using common chlorination agents SOCl₂ or POCl₃ led either to significant de-protected side product or require long temperatures and high reaction times. These chlorinating agents are therefore not suitable for high scale production of Melflufen. That was also confirmed as described in Comparative examples 1 or 2.

There is therefore a need for improved process for preparation of Melflufen suitable for high scale production.

### BRIEF DESCRIPTION OF THE INVENTION

The present invention relates for preparing Melflufen, compound of formula (1) or a salt thereof, 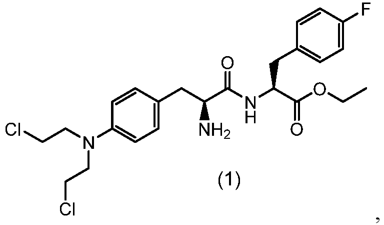 the process comprising:
a. Reacting a compound of formula (2) with compound of formula (3) to prepare compound of formula (4); 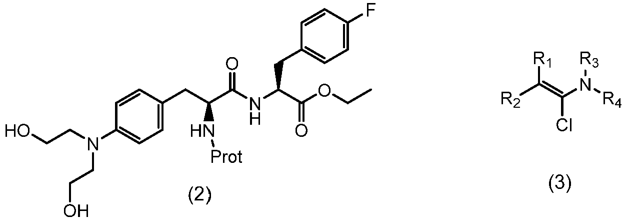 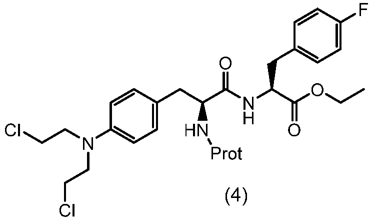
   Prot means nitrogen protective group;
   R₁, R₂, R₃, R₄ means C₁-C₃ alkyl;
b. Converting compound of formula (4) into Melflufen, compound of formula (1) or a salt thereof.

The presented invention further relates to intermediates used in the process and solid forms thereof.

### BRIEF DESCRIPTION OF THE DRAWINGS

Figure 1 depicts the X-Ray Powder Diffractogram (XRPD) of Form A of compound of formula (2A) prepared according to Example 3 or 4.
Figure 2 depicts the X-Ray Powder Diffractogram (XRPD) of Form B of compound of formula (6A) prepared according to Example 4.

### DETAILED DESCRIPTION OF THE INVENTION

The present invention relates for preparing Melflufen, compound of formula (1) or a salt thereof, 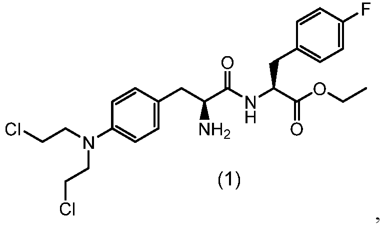 the process comprising:
a. Reacting a compound of formula (2) with compound of formula (3) to prepare compound of formula (4), 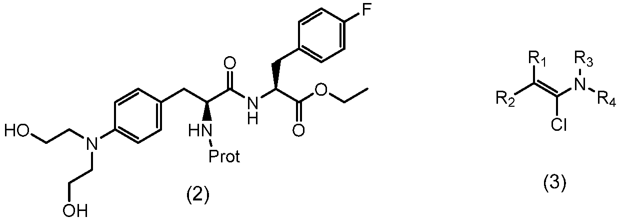 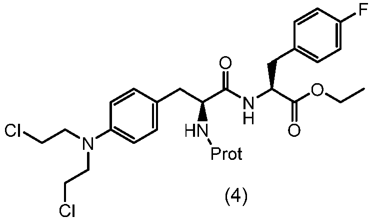
   Prot means nitrogen protective group
   R₁, R₂, R₃, R₄ means C₁-C₃ alkyl
b. Converting compound of formula (4) into Melflufen, compound of formula (1) or a salt thereof.

The salt of compound of formula (1) can be selected from for example hydrochloride or hydrobromide or methane sulfonate or ethane sulfonate or benzene sulfonate or toluene sulfonate or sulphate or hydrogen sulphate or fluoroacetate such as trifluoroacetate or fluoropropionate, preferably it is hydrochloride. Prot means a protective group that can be selected for example from tert-butyl oxycarbonyl (Boc), methyl oxycarbonyl, ethyl oxycarbonyl, 9- fluorenylmethyl oxycarbonyl (Fmoc), benzyl oxycarbonyl (Cbz), p-methoxybenzyl oxycarbonyl (Moz), 1-adamantyl oxycarbonyl (Adoc), p-bromobenzyl oxycarbonyl, trifluoroacetyl, chloroacetyl, phenylacetyl, benzacetyl, p-toluenesulfonyl (tosyl, Ts), 2-nitrobenzenesulfonyl (Nps), t-butylsulfonyl (Bus), 2- or 4-nitrobenzenesulfonyl (Nosyl), 2,4-dinitronenzesulfonyl (DNs), and 2-naphthalenesulfonyl, preferably it is tert-butyl oxycarbonyl (Boc).

R₁, R₂, R₃, R₄ in compound of formula (3) can be selected from methyl or ethyl or propyl or isopropyl, preferebly R₁, R₂, R₃, R₄ are methyl, i.e. compound of formula (3) is preferably compound of formula (3A),

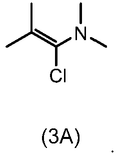

We have surprisingly found that use of compound of formula (3), preferably compound of formula (3A) provides compound of formula (4), preferably compound of formula (4A), in high yield and purity. Also the work up of the reaction mixture is easier comparing to work up of the mixture when the SOCl₂ or POCl₃ is used. Compound of formula (3), preferably of formula (3A), can be used at low temperature and is suitable for high scale production of Melflufen or a salt thereof.

The step a. can be performed under a protective atmosphere such as nitrogen or argon, in a solvent selected for example from acetonitrile or dimethylformamide or dichloromethane or trichloromethane or an aromatic solvent such as toluene or benzene or an ether such as dimethyl ether or diethyl ether or iso-propyl ether or tetrahydrofurane or 2-methyl tetrahydrofurane or an ester such as methyl acetate or ethyl acetate or isopropyl acetate. Acetonitrile is preferably used.

Compound of formula (2) is mixed with the solvent. The concentration of compound of formula (2) in the solvent can be between 0.04 g/ml and 0.16 g/ml, preferably it is between 0.04 g/ml and 0.07 g/ml. The mixture is cooled to a temperature between -10°C and 10°C, preferably between 0°C and 5°C. To the mixture compound of formula (3) is added. The molar ratio between compound of formula (2) and compound of formula (3) can be between 1:2 and 1:3, preferably between 1:2.4 and 1:2.7. Compound of formula (3) can be added in the course of between 5 and 30 minutes. The mixture is then stirred at the temperature between -10°C and 10°C, preferably between 0°C and 5°C, for between 5 and 30 minutes. The mixture was heated to a temperature between 20°C and 30°C, preferably between 20°C and 25°C and stirred at this temperature for between 3 and 24 hours. The reaction progress can be monitored by any suitable analytical technique, e.g. by HPLC or GC. After the reaction is finished the mixture can be optionally cooled to a temperature between -5°C and 5°C. To the mixture water is added to precipitate solid compound of formula (4). Volume ratio between added water and the solvent used in step a. can be between 1:2 and 1:3. The mixture is stirred for between 10 and 60 minutes and solid mass is filtered off. Obtained solid can be optionally dried.

Compound of formula (3), preferably compound of formula (3A) is commercially available.

Compound of formula (4) can be transformed to Melflufen, compound of formula (1) or a salt thereof (step b.) for example by using an acid such as HCl or HBr or methane sulfonic acid or ethane sulfonic acid or benzene sulfonic acid or toluene sulfonic acid or H₂SO₄ or a fluoroacetic acid such as trifluoroacetic acid or fluoropropionic acid, preferably HCl is used. Compound of formula (4) can be also transformed into Melflufen by using hydrogenation in a presence of a catalyst.

Compound of formula (4) can be deprotected by any of the process disclosed in the prior art or for example by a process comprising:
I. Mixing compound of formula (4), preferably compound of formula (4A) and a suitable solvent; 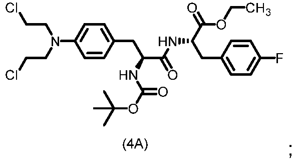
II. Adding the acid;
III. Isolating the compound of formula (1) or a salt thereof.

Compound of formula (4), preferably compound of formula (4A) is mixed with a suitable solvent such as acetonitrile or an alcohol, for example methanol or ethanol or propanol or isopropanol or ethylacetate or tetrahydrofurane or acetone or dichloromethane or trichloromethane or an ether, preferably acetonitrile is used. The concentration of compound of formula (4), preferably compound of formula (4A), in the solvent can be between 0.06 and 1.1 g/ml.

To the mixture an acid such as HCl or HBr or methane sulfonic acid or ethane sulfonic acid or benzene sulfonic acid or toluene sulfonic acid or H₂SO₄ or fluoroacetic acid such as trifluoroacetic acid or fluoropropionic acid, preferably HCl, is added. The molar ratio between used acid and the compound of formula (4) can be between 4:1 and 10:1, preferably it is between 5:1 and 7:1. The acid can optionally be used be used in form of a solution in a suitable solvent, for example cyclopentyl methyl ether or an alcohol such as ethanol or propanol or isopropanol or ethylacetate or tetrahydrofurane or acetone. Preferably HCl is be used as a solution in as cyclopentyl methyl ether or ethyl acetate. In comparison with the solvents disclosed in the prior art, cyclopentyl methyl ether or ethyl acetate has following advantages:
- It improves the stability of the acid;
- When a solution of the acid in an alcohol is used, genotoxic alkyl chlorides are formed as a side product in the reaction. These side products are not formed when cyclopentyl methyl ether or ethyl acetate is used as a solvent for the acid.

The mixture is then heated to a temperature between 35°C and 50°C and stirred at this temperature for between 1 and 5 hours. The mixture is evaporated to dryness. The rest is dissolved in ethanol at 75-85°C. The volume ratio between ethanol and the solvent used in step I to dissolve compound of formula (4) can be between 1:1 and 1:2. The mixture is then cooled to a temperature between 20°C and 25°C and stirred at this temperature for between 30 and 120 minutes to provide a suspension. To the mixture an antisolvent (i.e. a solvent that poorly dissolves Melflufen salt) is added. The volume ratio between ethanol and the antisolvent can be between 1:10 and 1:16. The mixture is cooled to a temperature between - 5°C and 5°C and stirred for between 1 and 3 hours. Obtained suspension is filtered off and filter cake is washed with the antisolvent and optionally dried.

The process for preparation of compound of formula (4) by reacting compound of formula (2) and compound of formula (3) is a two-step process depicted in following scheme:

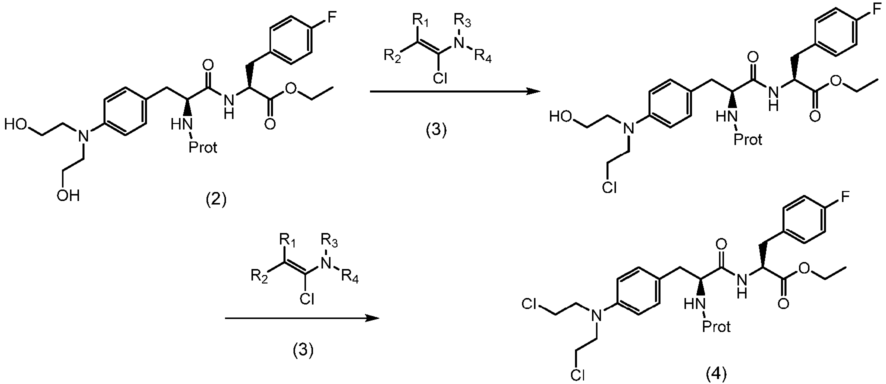

Another embodiment of the presented invention is therefore compound of following formula and use thereof for preparing of Melflufen or a salt thereof:

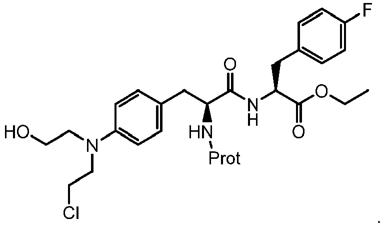

Compound of formula (2) can be prepared by a process comprising:
I. Reacting compound of formula (5) with a base in a suitable solvent to provide compound of formula (6) or a salt thereof, 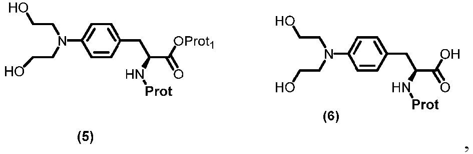
   Prot means nitrogen protective group
   Prot₁ means an oxygen protective group, preferably selected from C₁-C₆ alkyl;
II. Reacting compound of formula (6) or a salt thereof with compound of formula (7) or a salt thereof in a presence of suitable coupling agent to provide compound of formula (2), 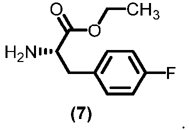 Nitrogen protective group (Prot) can be selected from for example tert-butyl oxycarbonyl (Boc), methyl oxycarbonyl, ethyl oxycarbonyl, 9- fluorenylmethyl oxycarbonyl (Fmoc), benzyl oxycarbonyl (Cbz), p-methoxybenzyl oxycarbonyl (Moz), 1-adamantyl oxycarbonyl (Adoc), p-bromobenzyl oxycarbonyl, trifluoroacetyl, chloroacetyl, phenylacetyl, benzacetyl, p-toluenesulfonyl (tosyl, Ts), 2-nitrobenzenesulfonyl (Nps), t-butylsulfonyl (Bus), 2- or 4-nitrobenzenesulfonyl (Nosyl), 2,4-dinitronenzesulfonyl (DNs), and 2-naphthalenesulfonyl, preferably it is tert-butyl oxycarbonyl (Boc). Compounds of formulas (5), (6) and (2) are preferably compounds of formulas (5A), (6A) and (2A),

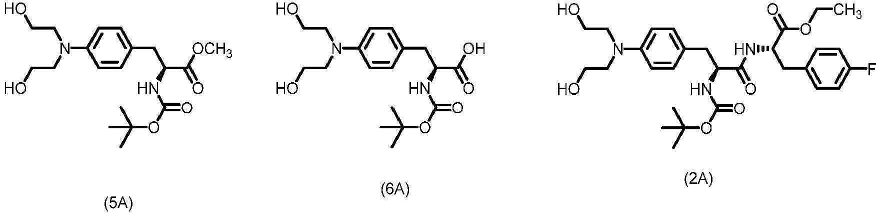

Compound of formula (5) is reacted with a suitable base in a suitable solvent (step I.) to provide compound of formula (6) or a salt thereof. Suitable base be selected from a hydroxide such as LiOH or NaOH or KOH or a combination of LiBr and an amine such as triethyl amine or diisopropyl ethyl amine in a presence of water. Suitable solvent can be selected from dimethylformamide or an alcohol such as MeOH or EtOH or dimethylformamide in a mixture with water or the alcohol in a mixture with water. Preferably a mixture of dimethylformamide (DMF) with water or a mixture of the alcohol with water is used. The volume ratio between dimethylformamide and water can be between 1.8:1 and 3:1, preferably it is between 2:1 and 2.5:1. The volume ration between the alcohol and water can be between 3.8:1 and 5:1, preferably it is between 3.9:1 and 4.5:1.

The concentration of compound of formula (5) in the solvent or solvent mixture can be between 0.1 g/ml and 0.8 g/ml. The concentration of the base in the solvent can be between 0.02 g/ml and 0.1 g/ml. The base can be used as a solid or in a form of a solution in a suitable solvent, for example water. The molar ratio between the base and the compound of formula (5) can be between 1:1 and 5:1, preferably between 1:1 and 2:1. Compound of formula (5) is mixed with the solvent or solvent mixture (solvent1). To the mixture the base is added. The base can be optionally added in parts, for example in 2 or 3 or 4 or 5 or 6 parts. The mixture is then stirred for between 1 and 5 hours at 20 - 35°C. The mixture can be optionally cooled to a temperature between 0°C and 10°C. pH of the mixture is set to between 2 and 4.5, preferably to 3.5 for example with citric acid (10% w/w queous solution) or with aqueous solution of HCl. The aqueous solution of the acid can be added in several parts, for example in 2 or 3 or 4 or 5 or 6 parts, more preferably it is added drop-wise. Obtained reaction mixture can be used in subsequent step for preparation of compound of formula (2) or compound of formula (6) can be isolated in a solid form.

Compound of formula (6) can be isolated by a process comprising adding a solvent (solvent_added) to the mixture. The solvent (solvent_added) can be for example toluene or an alkyl acetate such as ethyl acetate or iso-propyl acetate or 2-methyl tetrahydrofurane or dichloromethane or chloroform or trichloroethylene. The volume ratio between the added solvent (i.e. solvent_added) and the solvent used for mixing with compound of formula (5) (i.e. solvent1) can be between 1.4:1 and 2:1.

In the case when toluene is used as solvent_added, after addition of toluene the mixture is stirred at a temperature between 0°C and 10°C for between 10 and 20 hours to obtain a suspension. The suspension is filtered off and obtained solid is dried to provide a solid form of compound of formula (6).

In the case of compound of formula (6), where Prot means tert-butyl oxycarbonyl (Boc), i.e. compound of formula (6A), 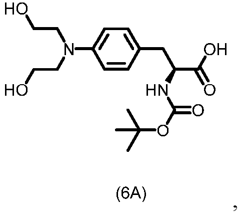 the obtained solid of compound of formula (6A), Form B, can be characterized by XRPD pattern having 2θ values 13.0°, 14.9° and 19.2° 2θ (± 0.2 degrees 2θ). The form can be also characterized by XRPD pattern having 2θ values 11.8°, 13.0°, 14.9°, 19.2° and 20.6° 2θ (± 0.2 degrees 2θ). The Form B can be further characterized by XRPD 2θ values (± 0.2 degrees 2θ) stated in following table:

| **Angle (2-Theta °)** | **Intensity (%)** | **Angle (2-Theta °)** | **Intensity (%)** |
|---|---|---|---|
| 6.4 | 4.2 | 22.5 | 5.2 |
| 8.7 | 1.6 | 22.6 | 7.1 |
| 10.8 | 8.7 | 23.5 | 10.9 |
| 11.3 | 8.3 | 24.1 | 9.1 |
| 11.6 | 2.3 | 24.5 | 10.3 |
| 11.8 | 25.1 | 24.8 | 7.3 |
| 13.0 | 32.4 | 25.4 | 16.4 |
| 13.8 | 20.6 | 25.7 | 4.2 |
| 14.9 | 46.3 | 26.0 | 2.2 |
| 15.1 | 5.9 | 26.3 | 2.5 |
| 16.1 | 2.8 | 26.7 | 2.6 |
| 17.6 | 10.1 | 27.6 | 4.1 |
| 17.8 | 6.0 | 27.8 | 4.9 |
| 19.2 | 100.0 | 29.2 | 7.1 |
| 19.6 | 3.7 | 29.5 | 3.7 |
| 19.8 | 3.2 | 30.0 | 7.1 |
| 20.1 | 21.3 | 31.0 | 4.3 |
| 20.3 | 10.6 | 31.5 | 2.3 |
| 20.6 | 29.0 | 31.8 | 1.9 |
| 20.8 | 5.2 | 32.4 | 1.9 |
| 21.3 | 2.6 | 32.9 | 4.3 |
| 21.6 | 4.3 | 33.7 | 2.4 |
| 22.0 | 9.8 | 34.4 | 2.8 |

The Form B can be also characterized by XRPD pattern depicted in Figure 2.

In the case when the added solvent (solvent_added) is different from toluene, i.e. selected for example from an alkyl acetate such as ethylacetate or iso-propyl acetate or 2-methyl tetrahydrofurane or dichloromethane or chloroform or trichloroethylene, the solid for can be isolated by using the following process. After addition of the solvent (solvent_added), the mixture is stirred for between 15 and 60 minutes and the layers are separated. The layer comprising added solvent (solvent_added) is evaporated to dryness. To the rest a second amount of solvent (solvent_added) was added. The volume ratio between solvent (solvent_added) and the solvent used for mixing with compound of formula (5) (i.e.solvent1) can be between 1.6:1 and 2:1. The mixture is then stirred for between 15 and 60 minutes and the layers are separated. The layer comprising added solvent (solvent_added) is evaporated to dryness to provide compound of formula (6).

Compound of formula (5) is commercially available or can be prepared from a compound of formula (8) by a process disclosed in prior art or by a process comprising reacting compound of formula (8) with ethylene oxide or with a compound of formula (9), for example by a process described in Example 1,

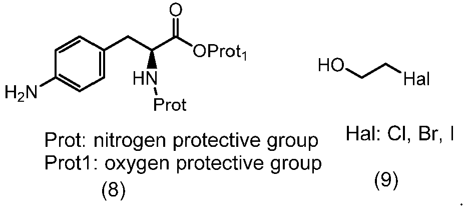

In step II. compound of formula (6), preferably of compound formula (6A), is reacted with compound of formula (7) or a salt thereof in a presence of a base and a coupling agent to provide compound of formula (2), preferably of formula (2A). Compound of formula (7) is preferably in form of a salt, more preferably in a form of HCl salt. The reaction is performed in a suitable solvent. The suitable solvent can be selected from for example from acetonitrile or ethyl acetate or dimethylsulfoxide or acetone or tetrahydrofurane or 2-methyltetrahydrofurane or dioxane or an alcohol such as methanol or ethanol or propanol or dichloromethane or dimethylformamide or cyclopentyl methyl ester or acetone or water and combination thereof, preferably dimethylformamide in combination with water or acetonitrile is used. In the case a combination of a solvent with water is used, the volume ratio between the solvent and water can be between 3:1 and 6:1, preferably it is between 4:1 and 5:1.

As a coupling agent for example propylphosphonic anhydride (T3P), 2-chloro-4,6-dimethoxy-1,3,5-triazine (CDMT), dicyclohexylcarbodiimide (DCC), diisopropylcarbodiimide (DIC), ethyl-(N',N'- dimethylamino)propylcarbodiimide hydrochloride (EDC); phosphonium-based reagents, for example (benzotriazol-1-yloxy)tris(dimethylamino)phosphonium hexafluorophosphate (BOP), benzotriazol-1-yl-oxytripyrrolidinophosphonium hexafluorophosphate (PyBOP); aminium-based reagents, for example N,N,N',N'-tetramethyl-O-(1H-benzotriazol-1-yl)uronium hexafluorophosphate (HBTU), 1-[Bis(dimethylamino)methylene]-1H-1,2,3- triazolo[4,5-b]pyridinium 3-oxid hexafluorophosphate (HATU), O-(benzotriazol-1-yl)- N,N,N',N'-tetramethyluronium tetrafluoroborate (TBTU), O-(6-chlorobenzotriazol-1-yl)- N,N,N',N'-tetramethyluronium hexafluorophosphate (HCTU), O-(3,4-dihydro-4-oxo-1,2,3- benzotriazine-3-yl)-N,N,N',N'-tetramethyluronium tetrafluoroborate (TDBTU); immonium aminium-based reagents, for example (1H-benzotriazol-1-yloxy)-N,N- dimethylmethaniminium hexachloroantimonate (BOMI), 5-(1H-benzotriazol-1-yloxy)-3,4- dihydro-1 -methyl 2H-pyrrolium hexachloroantimonate (BDMP) and 5-(7-azabenzotriazol-1-yloxy)-3,4-dihydro-1-methyl 2H-pyrrolium hexachloroantimonate (AOMP); agents generating acids chlorides, for example thionyl chloride, phosphorus pentachloride, triphosgene, triazines (e.g. cyanuric chlorides, cyanuric fluoride, and derivatives thereof), tetramethylfluoroformamidinium hexafluorophosphate (TFFH), bis(tetramethylene)fluoroformamidinium (BTFFH), and 1,3-dimethyl-2-f uoro-4,5-dihydro- lH-imidazolium hexafluorophosphate (DFIH); or other coupling reagents, for example 3- (diethylphosphoryloxy)-1,2,3-benzotriazin-4(3H)-one (DEPBT), carbonyldilmidazole (CDI), and N-Ethoxycarbonyl-2-ethoxy-l,2-dihydroquinoline (EEDQ). Preferably propylphosphonic anhydride (T3P) or 2-chloro-4,6-dimethoxy-1,3,5-triazine (CDMT) is used, more preferably 2-chloro-4,6-dimethoxy-1,3,5-triazine (CDMT) is used.

Suitable base can be selected from N-methyl morpholine or an amine such as trimethyl amine or triethyl amine or diisopropyl ethyl amine (DIPEA) or a carbonate such as sodium carbonate or potassium carbonate or a hydrogen carbonate such as sodium hydrogen carbonate or potassium hydrogen carbonate or a hydroxide such as sodium hydroxide or potassium hydroxide. The base is preferably N-methyl morpholine.

For the preparation of compound of formula (2), preferably formula (2A), the reaction mixture from the previous step comprising compound of formula (6), preferably compound of formula (6A), can be used. Compound of formula (2) can be also prepared from isolated solid compound of formula (6), preferably compound of formula (6A). In the second case, compound of formula (6) is mixed with the solvent or a mixture of solvents, preferably with acetonitrile or with a mixture of dimethylformamide with water. The concentration of compound of formula (6) in the solvent can be between 0.04 g/ml and 0.15 g/ml, preferably it is between 0.1 and 0.15 g/ml. Compound of formula (7), preferably in form of HCl salt, is added to the mixture or the reaction mixture from the previous step. The molar ratio between compound of formula (6) and compound of formula (7) can be between 1:1.1 and 1:1.5. To the mixture the base is added. The molar ratio between the base and the compound of formula (6) can be between 1:2 and 1:5, preferably it is between 1:2.4 and 1:3.3. The mixture is stirred at 20-25°C for between 5 and 30 minutes. To the mixture the coupling agent is added in the course of between 5 and 60 minutes. The molar ratio between the coupling agent and the compound of formula (6) can be between 1.1:1 and 2:1, preferably it is between 1.1:1 and 1.5:1. The mixture is then stirred at 20-25°C for between 2 and 10 hours. The reaction progress can be monitored by any suitable analytical technique, e.g. by HPLC or GC.

Compound of formula (2) can be also prepared by a process comprising mixing the coupling agent with a solvent or a solvent mixture. The concentration of the coupling agent in the solvent or the solvent mixture can be between 0.04 g/ml and 0.15 g/ml. The molar ration between the coupling agent and the compound of formula (6) can be between 1.1:1 and 2:1, preferably it is between 1.1:1and 1.5:1. To the mixture the base is added. The molar ratio between the base and the compound of formula (6) can be between 1:2 and 1:5, preferably it is between 1:2.4 and 1:3.3. The mixture is the stirred for between 5 and 60 minutes. To the mixture compound of formula (7), preferably in a form of HCl salt is added. The molar ratio between compound of formula (6) and compound of formula (7) can be between 1:1.1 and 1:1.5. The mixture is stirred for between 5 and 60 minutes. To the mixture compound of formula (6) is added either in form of isolated solid or in a form of reaction mixture from the previous step. The mixture is then stirred at 20-25°C for between 2 and 10 hours. The reaction progress can be monitored by any suitable analytical technique, e.g. by HPLC or GC.

After the reaction is finished the reaction mixture can be processed either by:
i. Addition of water and a water immiscible solvent, separation of the phases, drying of the organic phase and evaporating of the organic phase; or
ii. Addition of a mixture of acetonitrile and water and isolation of a solid form of compound of formula (2), preferably of compound of formula (2A).

In the case i. the water immiscible solvent can be selected for example from an alkylacetate, such as methyl acetate or ethyl acetate or propyl acetate or iso-propyl acetate or dichloromethane or chloroform or trichloroethylene, preferably ethyl acetate is used. The volume ratio between water and the water immiscible solvent can be between 1:0.8 and 1:1.3. The volume ratio between added mixture of water and the water immiscible solvent and the solvent used to dissolve compound of formula (6) can be between 1:0.5 and 1:1. The mixture is then stirred for between 5 and 30 minutes. The layers are separated and to the organic layer a saturated solution of a hydrogen carbonate such as NaHCO₃ or KHCO₃ is added. The volume ratio between the organic layer and added saturated solution of the hydrogen carbonate can be between 1:1 and 1:2. The mixture is stirred for between 5 and 30 minutes and the layers are separated. The organic layer is dried, for example using MgSO₄. The mixture is then filtrated off and the filtrate is evaporated to dryness to provide compound of formula (2), preferably compound of formula (2A).

In the case ii., the volume ratio acetonitrile: water can be between 1:1.5 and 1:3, preferably between 1:1.5 and 1:2. The volume ratio between the added mixture water and acetonitrile and the solvent or a solvent mixture used to dissolve the compound of formula (6) can be between 2:1 and 3:1, preferably it is between 2:1 and 2.5:1. The mixture of acetonitrile and water can be added in parts, for example in 2 or 3 or 4 or 5 or 6 or 7 parts, more preferably it is added drop-wise in the course of between 10 and 60 minutes. The mixture is then concentrated at between 33 and 35°C to approximately 1/5 of the original volume to obtain a suspension. The suspension is cooled to between 20°C and 25°C and stirred at this temperature for between 1 and 3 hours. Obtained solid is filtered off and dried. In the case of compound of formula (2), where Prot means tert-butyl oxycarbonyl (Boc), i.e. compound of formula (2A), 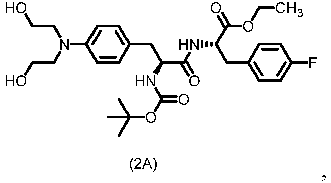 the obtained solid of compound of formula (2A), Form A, can be characterized by XRPD pattern having 2θ values 5.7°, 17.4° and 19.0° 2θ (± 0.2 degrees 2θ). The form can be also characterized by XRPD pattern having 2θ values 5.7°, 8.5°, 17.4°, 19.0° and 21.6° 2θ (± 0.2 degrees 2θ). The Form A can be further characterized by XRPD 2θ values (± 0.2 degrees 2θ) stated in following table:

| **Angle (2-Theta °)** | **Intensity (%)** | **Angle (2-Theta °)** | **Intensity (%)** |
|---|---|---|---|
| 2.8 | 3.8 | 20.0 | 18.4 |
| 5.7 | 100.0 | 20.8 | 22.4 |
| 6.4 | 3.5 | 21.0 | 20.0 |
| 7.4 | 6.1 | 21.6 | 19.8 |
| 8.5 | 19.2 | 22.2 | 13.8 |
| 9.5 | 16.5 | 22.8 | 12.4 |
| 11.3 | 8.7 | 23.6 | 5.7 |
| 11.8 | 6.1 | 25.8 | 6.9 |
| 14.2 | 8.0 | 27.3 | 6.7 |
| 17.1 | 6.6 | 28.7 | 7.1 |
| 17.4 | 24.8 | 32.0 | 5.6 |
| 19.0 | 65.6 | | |

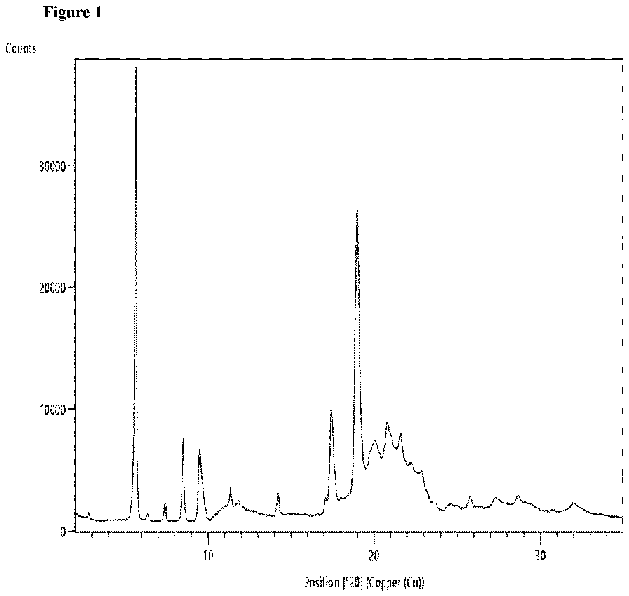

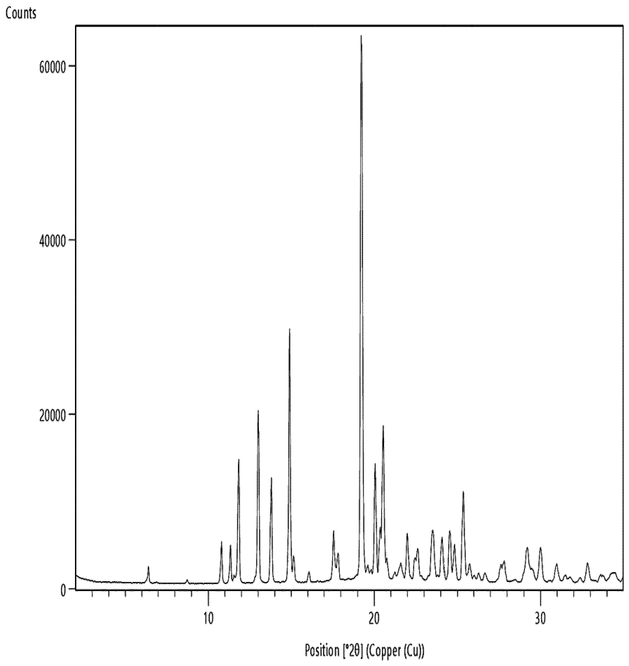

The Form A can be also characterized by XRPD pattern depicted in Figure 1.

The invention will be further described with reference to the following examples.

### EXAMPLES

XRPD spectrum of solid compounds was obtained using the following measurement conditions:
Panalytical Empyrean diffractometer with Θ/2Θ geometry (transmition mode), equipped with a PixCell 3D detector;

| | |
|---|---|
| Start angle (2θ): | 2.0° |
| End angle (2θ): | 35.0° |
| Step size: | 0.026° |
| Scan speed: | 0.0955 °/seconds |
| Radiation type: | Cu |
| Radiation wavelengths: | 1.5406Å (Kα1), primary monochromator used |
| Divergence slit: | 1/2° |
| Antiscatter slit: | 1/2° |
| Soller slit: | 0.02 rad |
| Detector slit: | 7.5 mm |
| Rotation speed: | 30 rpm |

### Example 1: Preparation of methyl (S)-3-(4-(bis(2-hydroxyethyl)amino)phenyl)-2-((tert-butoxycarbonyl)amino)propanoate

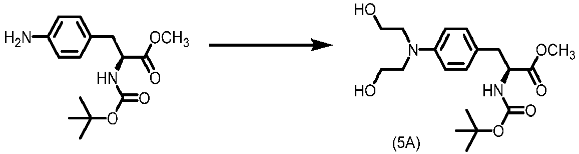

0.6 g of methyl (S)-3-(4-aminophenyl)-2-((tert-butoxycarbonyl)amino)propanoate was dissolved in 6 ml of in acetonitrile. 1.222 g of sodium iodide, 0.648 g of sodium carbonate and 0.579 ml of 2-bromoethan-1-ol were added and then the mixture was heated to 80 °C. The mixture was stirred at 80 °C under argon for 47.5 hours. The mixture was cooled to room temperature (20-25°C), filtered and the filter cake was washed with 2 ml of acetonitrile. The mother liquor was evaporated (60 °C, 120 mbar).

The residue was dissolved in 6 ml of ethyl acetate. 6 ml of water was added and the mixture was stirred for 5 min. The layers were separated. Organic layer was dried and evaporated to give 0.71 g of mass. The rest was dissolved in 6 ml of ethyl acetate. The mixture was twice extracted with 6 ml of 10% solution of sodium thiosulphate and then with 6 ml of water. The layers were separated. Organic layer was dried and evaporated to give 0.30 g of the product.

### Example 2: Preparation of ethyl (S)-2-((S)-3-(4-(bis(2-hydroxyethyl)amino)phenyl)-2-((tert-butoxycarbonyl)amino)propanamido)-3-(4-fluorophenyl)propanoate (compound of formula (2A))

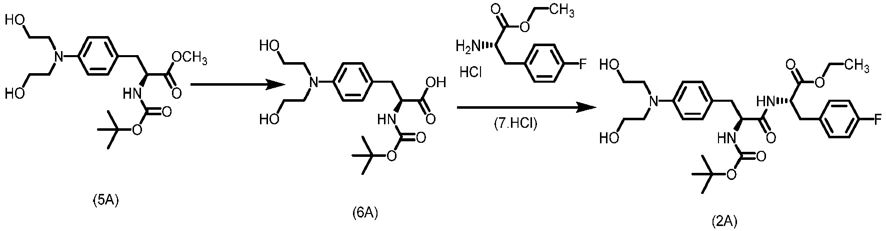

5.3 g of compound of formula (5A) was dissolved in 40 ml of methanol. Solution of 1.163 g of LiOH·H₂O in 10 ml of water was added. Mixture was stirred for 1 hour and 1.163 g of LiOH·H₂O was added to reaction mixture. Mixture was stirred for 1 hour. Mixture was acidified with 10% (w/w) citric acid to pH 3.5. To the mixture 70 ml of ethyl acetate was added. Mixture was stirred for 20 minutes and layers were separated. Organic layer was evaporated on the rotary evaporator (300-30 mbar, 55°C). To the rest 70 ml of ethyl acetate was added and mixture was stirred for 20 minutes. Layers were separated. Organic layer was evaporated to dryness (300-30 mbar) to provide 4.55 g of compound of formula (6A) in 86 % yield, and 96 % purity (HPLC).

2 g of compound of formula (6A) was mixed with 30 ml of acetonitrile. To the mixture 1.479 g of compound of formula (7.HCl) was added at 20-25°C. 1.79 ml of N-methyl morpholine was added and the mixture was stirred at 20-25°C for 5 minutes. 4.15 g of AllessanCAP (1-Propanephosphonic anhydride, 50% solution in acetonitrile) in acetonitrile was added via syringe over 5 minutes and resulting solution was stirred at 20-25°C for 3 hours. To the mixture 20 ml of water and 20 ml of ethyl acetate were added and the mixture was stirred for 10 minutes. Layers were separated and organic layer was washed with 20 ml of saturated NaHCO₃ solution for 5 minutes. Layers were separated. Organic layer was dried with MgSO₄, filtered, and evaporated to dryness (300-50 mbar, 50°C) providing 3 g of compound of formula (2A) in 91 % yield and 93 % purity (HPLC).

### Example 3: Preparation of ethyl (S)-2-((S)-3-(4-(bis(2-hydroxyethyl)amino)phenyl)-2-((tert-butoxycarbonyl)amino)propanamido)-3-(4-fluorophenyl)propanoate (compound of formula (2A))

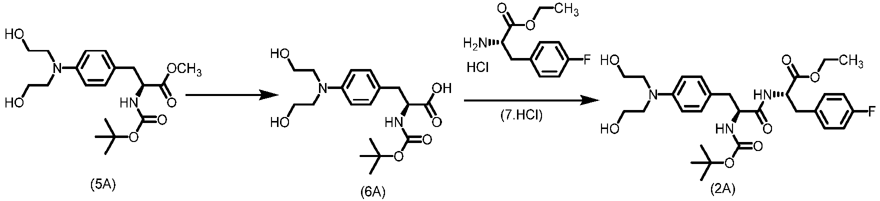

100 g of methyl (S)-3-(4-(bis(2-hydroxyethyl)amino)phenyl)-2-((tert-butoxycarbonyl)amino)propanoate (compound of formula (5A)) was mixed with 100 g of dimethylformamide (DMF) and 50 g of water to dissolve the compound of formula (5A). 12.05 g of LiOH.H₂O was added and the solution was stirred at 30 °C for 3 hours. The solution was cooled down to 5 °C and 29.1 g of 36% aq. solution of HCl diluted with 100 g of water was added dropwise in the course of 5 minutes to obtain reaction mixture (RM1).

52.8 g of 2-chloro-4,6-dimethoxy-1,3,5-triazine was dissolved in 325 g of dimethylformamide (DMF) under nitrogen atmosphere. The mixture was cooled down to -10 °C. 66.1 g of 4-Methylmorpholine was added and the mixture was stirred for 10 minutes. To the mixture 74.5 g of Ethyl (S)-2-amino-3-(4-fluorophenyl)propanoate hydrochloride (compound 7.HCl) was added followed by 200 g of dimethylformamide. The mixture was stirred for 5 minutes. To the mixture previously prepared reaction mixture (RM1) was added in the course of 1 minute.

The mixture was stirred for 2 hours at 22 °C. To the mixture 560 g of acetonitrile was added. To the mixture 1170 g of water was added dropwise in the course of 20 minutes. The mixture was distilled at 33-35 °C under vacuum (200 mbar) until the volume of the reaction mixture was reduced to approximately 625 mL. The suspension was cooled down to 22 °C and stirred for 1 hour. The product was filtered and washed with 250 g of water. The solid material was dried on the Buchner funnel for 1 hour and then in vacuo for 16 hours. 126.0 g (86 % yield) of Ethyl (S)-2-((S)-3-(4-(bis(2-hydroxyethyl)amino)phenyl)-2-((tert-butoxycarbonyl)amino)pro-panamido)-3-(4-fluorophenyl)propanoate was isolated as off-white powder with purity 99.5% (HPLC IN).

XRPD pattern of obtained solid corresponds to XRPD pattern depicted in Figure 1.

### Example 4: Example 3: Preparation of ethyl (S)-2-((S)-3-(4-(bis(2-hydroxyethyl)amino) phenyl)-2-((tert-butoxycarbonyl)amino)propanamido)-3-(4-fluorophenyl)propanoate (compound of formula (2A))

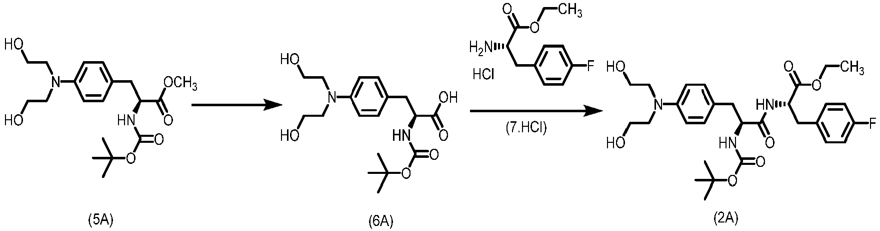

100 g of methyl (S)-3-(4-(bis(2-hydroxyethyl)amino)phenyl)-2-((tert-butoxycarbonyl)amino) propanoate (compound of formula (5A)) was mixed with 100 g of dimethylformamide and 50 g of water to dissolve compound of formula (5A). 12.05 g of LiOH.H₂O was added and the mixture was stirred at 30 °C for 3 hours. The solution was cooled down to 5 °C. A mixture of 29.1 g of 36% aq. solution of HCl and 300 g of water was added dropwise over 5 minutes. To the mixture 200 g of toluene was added dropwise over 30 minutes at 15 °C. The mixture was stirred at 5 °C for 16 hours. Obtained suspension was filtered off and obtained solid was washed with 200 g of water. Obtained solid material was dried in the Buchner funnel for 1 hour and then in vacuo for 16 hours to provide 77.2 g (80% yield) of (S)-3-(4-(bis(2-hydroxyethyl)amino)phenyl)-2-((tert-butoxycarbonyl)amino)propanoic acid in purity 99.7% (HPLC IN). XRPD of obtained solid corresponds to XRPD pattern depicted in Figure 2.

42.3 g of 2-chloro-4,6-dimethoxy-1,3,5-triazine was dissolved in 260 g of dimethylformamide under nitrogen atmosphere. The mixture was cooled to -10 °C. 53 g of 4-Methylmorpholine was added and the mixture was stirred for 10 minutes. 59.7 g of Ethyl (S)-2-amino-3-(4-fluorophenyl) propanoate hydrochloride (compound (7.HCl)) was added followed by 160 g of dimethylformamide (DMF). The mixture was stirred for 5 minutes. 77.2 g of (S)-3-(4-(bis(2-hydroxyethyl)amino) phenyl)-2-((tert-butoxycarbonyl)amino)propanoic acid was added followed by 80 g of dimethylformamide and 120 g of water.

The mixture was stirred for 2 hours at 22 °C. To the mixture 450 g of acetonitrile was added. 950 g of water was added dropwise over 20 minutes. The mixture was distilled at 33-35 °C under vacuum (200 mbar) until the volume of the reaction mixture was reduced to approximately 500 mL. The suspension was cooled down to 22 °C and stirred for 1 hour. The solid was filtered and washed with 250 g of water. The solid material was dried in the Buchner funnel for 1 hour and then in vacuo for 16 hours. 101.4 g (86 % yield) of Ethyl (S)-2-((S)-3-(4-(bis(2-hydroxyethyl)amino)phenyl)-2-((tert-butoxycarbonyl)amino)propanamido)-3-(4-fluorophenyl)propanoate was isolated with purity 99.7% (HPLC). XRPD pattern of obtained solid corresponds to XRPD pattern depicted in Figure 1.

### Example 5: Preparation of ethyl (S)-2-((S)-3-(4-(bis(2-chloroethyl)amino)phenyl)-2-((tert-butoxycarbonyl)amino)propanamido)-3-(4-fluorophenyl)propanoate (Compound of formula (4A))

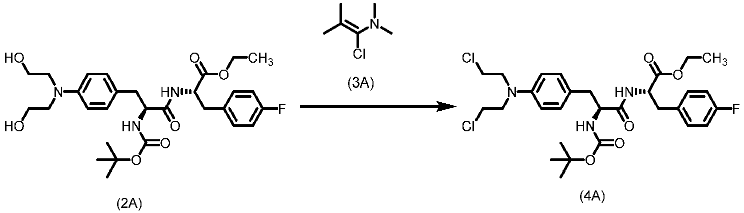

11.4 g of compound of formula (4A) was mixed with 230 ml of acetonitrile under argon atmosphere. The mixture was stirred at 20-25°C for 10 minutes to provide a solution. The mixture was cooled to -5°C-5°C (ice-bath) and 8.39 ml of 1-Chloro-N,N,2-Trimethylpropenylamine (Compound of formula (3A)) was gradually added via syringe over 10 minutes keeping reaction temperature below 5°C. The mixture was stirred at 0-5°C for next 10 minutes and then at 20-25°C for 17 hours. To the mixture 100 ml of water was added to provide a suspension. The suspension was then cooled to 0-5°C (ice bath) and stirred for 1 hour. The suspension was filtered off and filter cake was washed with 2x20 ml of chilled (0-5°C) acetonitrile. Wet product was air dried for 3 hours to provide 10 g of compound of formula (4A) in 82% yield and 99.3% purity (HPLC).

### Example 6: Preparation of HCl salt of Melflufen

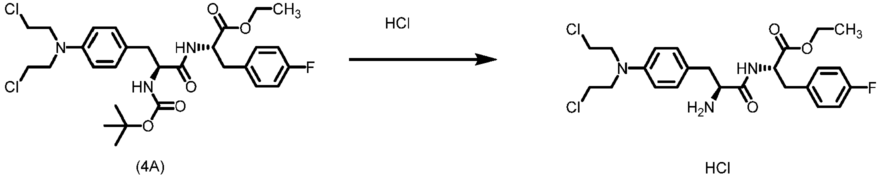

1 g of compound of formula (4A) was mixed with 12 ml of ethanol. 5.57 ml of hydrogen chloride in cyclopentyl methyl ether (CPME) (3M solution) was added at 20-25°C. The mixture was heated up to 40°C and stirred for 4 hours. Mixture was evaporated to dryness (300 - 150 mbar, 50°C) giving an oil. It was dissolved in 20 ml of ethanol and evaporated to dryness (300 - 80 mbar, 60°C). The rest was dissolved in 20 ml of ethanol at 60 °C and then stirred at 20-25°C for 12 hours giving a suspension. The mixture was cooled to 0-5°C, mixed with 10 ml of methyl tert-butyl ether and stirred for 2 hours. The solid was filtered off and the filter cake that was washed with 2x10 ml of methyl tert-butyl ether. Obtained solid was air-dried on filter for 2 h providing 0.75 g HCl salt of Melflufen in 86% yield and 99.4% purity (HPLC).

### Comparative example 1: Preparation of Melflufen using SOCl₂

0.060 g of compound (2A) was added to a reaction vial followed by 1 ml of dichloromethane and 0.078 ml of SOCl₂. Mixture was stirred at 40 °C for 22.5 h. Sample was evaporated in the stream of nitrogen to produce 62 mg of very dark green / black semi-solid containing 5.32 % of Melflufen by assay on external standard. Melflufen was not isolated. Because of low yield and purity of prepared Melflufen, the process is not suitable for high scale production.

### Comparative example 2: Preparation of melflufen using POCl₃

0.06 g of compound 2A was added to a reaction vial followed by 1 ml of acetonitrile and 0.033 ml of POCl₃. Mixture was stirred at 40 °C for 3 h. Sample was evaporated in the stream of nitrogen to produce 78 mg of a product containing 47.1 % of Melflufen by assay on external standard. Melflufen was not isolated.

Because of low yield and purity of prepared Melflufen, the process is not suitable for high scale production.

## Claims

1. A process for preparing Melflufen, compound of formula (1) or a salt thereof, 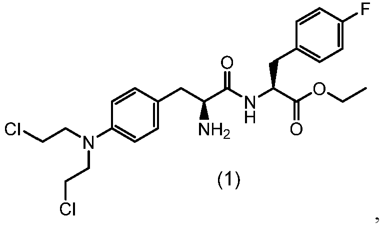 the process comprising:
a. Reacting a compound of formula (2) with compound of formula (3) to prepare compound of formula (4), 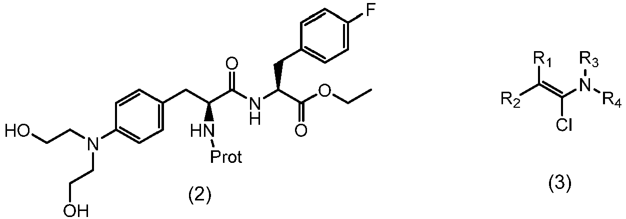 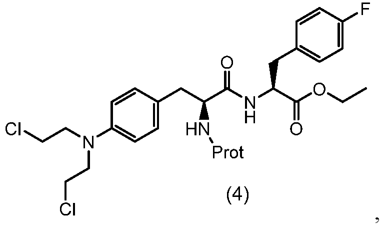
Prot means nitrogen protective group;
R₁, R₂, R₃, R₄ means C₁-C₃ alkyl;
b. Converting compound of formula (4) into Melflufen, compound of formula (1) or a salt thereof.

2. The process according to claim 1 wherein the Melflufen salt is hydrochloride salt.

3. The process according to claim 1 or 2 wherein the nitrogen protective group is tert-butyl oxycarbonyl (Boc).

4. The process according to any one of claims 1 to 3 wherein the compound of formula (3) is compound of formula (3A),

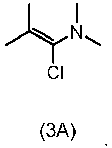

5. The process according to anyone of claims 1 to 4 wherein the step a. is performed in a solvent selected from acetonitrile or dimethylformamide or dichloromethane or trichloromethane or an aromatic solvent such as toluene or benzene or an ether such as dimethyl ether or diethyl ether or propyl ether or tetrahydrofurane or 2-methyl tetrahydrofurane or an ester such as methyl acetate or ethyl acetate or isopropyl acetate.

6. The process according to any one of claims 1 to 5 wherein the step a. is performed at a temperature between 15°C and 30°C.

7. The process according to any one of claims 1 to 6 wherein the step a. is performed for between 5 and 30 hours.

8. The process according to any one of claims 1 to 7 wherein the step b. comprises deprotecting compound of formula (4).

9. The process according to claim 8 wherein the compound of formula (4) is compound of formula (4A),

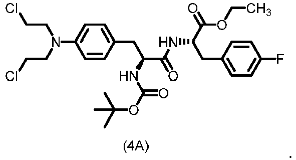

10. The process according to claims 8 or 9 wherein in step b. compound of formula (4) or compound of formula(4A) is deprotected with HCl dissolved in cyclopentyl methyl ether.

11. Compound of Formula, 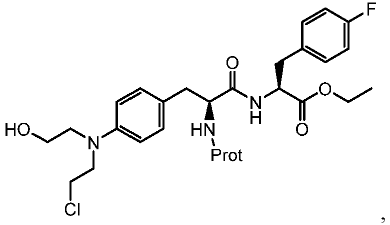 Prot means nitrogen protective group.

12. Use of compound according to claim 11 for preparation of Melflufen, compound of Formula (1) or a salt thereof.

13. A solid form of compound of formula (2A), Form A, 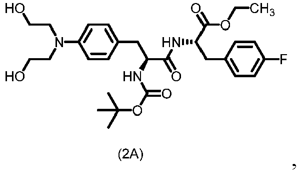 **characterized by** XRPD pattern having 2θ values 5.7°, 17.4° and 19.0° 2θ (± 0.2 degrees 2θ).

14. The solid form according to claim 13 **characterized by** XRPD pattern having 2θ values 5.7°, 8.5°, 17.4°, 19.0° and 21.6° 2θ (± 0.2 degrees 2θ).

15. A solid form of compound of formula (6A) Form B, **characterized by** XRPD pattern having 2θ values 13.0°, 14.9° and 19.2° 2θ (± 0.2 degrees 20),

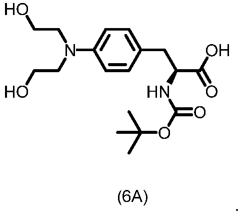

16. The solid form according to claim 15 **characterized by** XRPD pattern having 2θ values 11.8°, 13.0°, 14.9°, 19.2° and 20.6° 2θ (± 0.2 degrees 2θ).

## Patentansprüche

1. Verfahren zum Herstellen von Melflufen, einer Verbindung der Formel (1) oder eines Salzes davon, 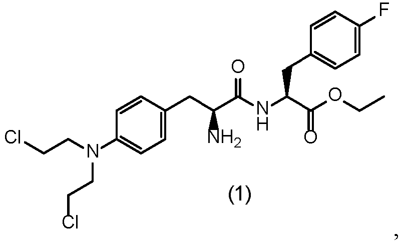 wobei das Verfahren umfasst:
a. Umsetzen einer Verbindung der Formel (2) mit einer Verbindung der Formel (3), um eine Verbindung der Formel (4) herzustellen, 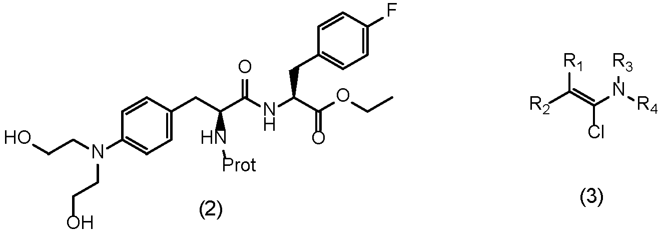 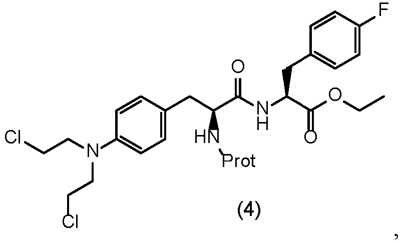
Prot bedeutet Stickstoffschutzgruppe;
R₁, R₂, R₃, R₄ bedeutet C₁-C₃-Alkyl;
b. Umwandeln einer Verbindung der Formel (4) zu Melflufen, einer Verbindung der Formel (1) oder eines Salzes davon.

2. Verfahren nach Anspruch 1, wobei das Melflufensalz ein Hydrochloridsalz ist.

3. Verfahren nach Anspruch 1 oder 2, wobei die Stickstoffschutzgruppe *tert-*Butyloxycarbonyl (Boc) ist.

4. Verfahren nach einem beliebigen der Ansprüche 1 bis 3, wobei die Verbindung der Formel (3) eine Verbindung der Formel (3A) ist,

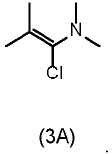

5. Verfahren nach einem beliebigen der Ansprüche 1 bis 4, wobei der Schritt a. in einem Lösungsmittel durchgeführt wird, das aus Acetonitril oder Dimethylformamid oder Dichlormethan oder Trichlormethan oder einem aromatischen Lösungsmittel wie Toluol oder Benzol oder einem Ether wie Dimethylether oder Diethylether oder Propylether oder Tetrahydrofuran oder 2-Methyltetrahydrofuran oder einem Ester wie Methylacetat oder Ethylacetat oder Isopropylacetat ausgewählt ist.

6. Verfahren nach einem beliebigen der Ansprüche 1 bis 5, wobei der Schritt a. bei einer Temperatur zwischen 15°C und 30°C durchgeführt wird.

7. Verfahren nach einem beliebigen der Ansprüche 1 bis 6, wobei der Schritt a. zwischen 5 und 30 Stunden lang durchgeführt wird.

8. Verfahren nach einem beliebigen der Ansprüche 1 bis 7, wobei der Schritt b. Entschützen einer Verbindung der Formel (4) umfasst.

9. Verfahren nach Anspruch 8, wobei die Verbindung der Formel (4) eine Verbindung der Formel (4A) ist,

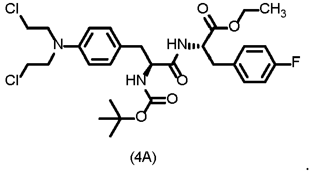

10. Verfahren nach Ansprüchen 8 oder 9, wobei in Schritt b. eine Verbindung der Formel (4) oder eine Verbindung der Formel (4A) mit in Cyclopentylmethylether gelöster HCl entschützt wird.

11. Verbindung der Formel 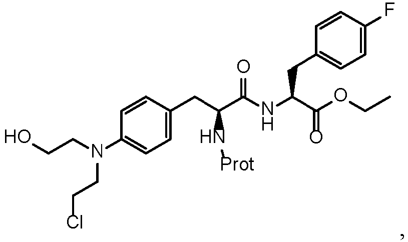 Prot bedeutet Stickstoffschutzgruppe.

12. Verwendung einer Verbindung gemäß Anspruch 11 zur Herstellung von Melflufen, einer Verbindung der Formel (1) oder eines Salzes davon.

13. Feste Form einer Verbindung der Formel (2A), Form A, 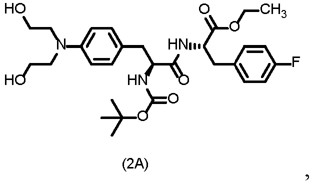 **gekennzeichnet durch** ein XRPD-Muster mit 2θ-Werten 5,7°, 17,4° und 19,0° 2θ (± 0,2 Grad 2θ).

14. Feste Form nach Anspruch 13, **gekennzeichnet durch** ein XRPD-Muster mit 2θ-Werten 5,7°, 8,5°, 17,4°, 19,0° und 21,6° 2θ (± 0,2 Grad 2θ).

15. Feste Form einer Verbindung der Formel (6A) Form B, **gekennzeichnet durch** ein XRPD-Muster mit 2θ Werten 13,0°, 14,9° und 19,2° 2θ (± 0,2 Grad 2θ),

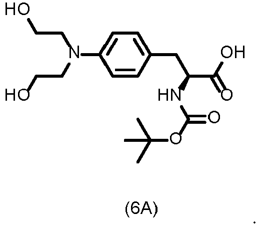

16. Feste Form nach Anspruch 15, **gekennzeichnet durch** ein XRPD-Muster mit 2θ-Werten 11,8°, 13,0°, 14,9°, 19,2° und 20,6° 2θ (± 0,2 Grad 2θ).

## Revendications

1. Un procédé de préparation de melflufen, composé de formule (1) ou d'un sel en dérivant : 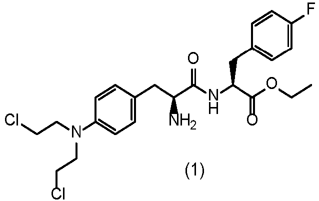 le procédé comprenant :
a. la réaction d'un composé de formule (2) avec un composé de formule (3) pour préparer un composé de formule (4), 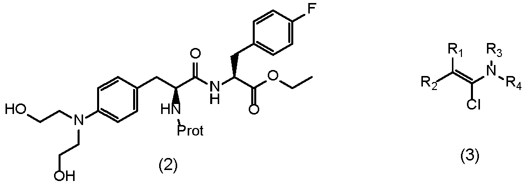 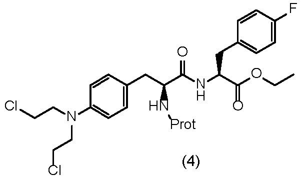
- Prot signifiant groupe protecteur d'azote
- R₁, R₂, R₃, R₄ signifiant alkyle en C₁-C₃ ;
b. la conversion du composé de formule (4) en melflufen, composé de formule (1) ou un sel en dérivant.

2. Le procédé selon la revendication 1, dans lequel le sel de melflufen est un sel de chlorhydrate.

3. Le procédé selon la revendication 1 ou 2, dans lequel le groupe protecteur de l'azote est le tert-butyloxycarbonyle (Boc).

4. Le procédé selon l'une quelconque des revendications 1 à 3, dans lequel le composé de formule (3) est un composé de formule (3A),

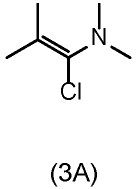

5. Le procédé selon l'une quelconque des revendications 1 à 4, dans lequel l'étape a. est réalisée dans un solvant choisi parmi l'acétonitrile ou le diméthylformamide ou le dichlorométhane ou le trichlorométhane ou un solvant aromatique tel que le toluène ou le benzène ou un éther tel que l'éther diméthylique ou l'éther diéthylique ou l'éther propylique ou le tétrahydrofurane ou le 2-méthyl tétrahydrofurane ou un ester tel que l'acétate de méthyle ou l'acétate d'éthyle ou l'acétate d'isopropyle.

6. Le procédé selon l'une quelconque des revendications 1 à 5, dans lequel l'étape a. est réalisée à une température comprise entre 15°C et 30°C.

7. Le procédé selon l'une quelconque des revendications 1 à 6, dans lequel l'étape a. est réalisée pendant une durée comprise entre 5 et 30 heures.

8. Le procédé selon l'une quelconque des revendications 1 à 7, dans lequel l'étape b. comprend la déprotection du composé de formule (4).

9. Le procédé selon la revendication 8, dans lequel le composé de formule (4) est un composé de formule (4A).

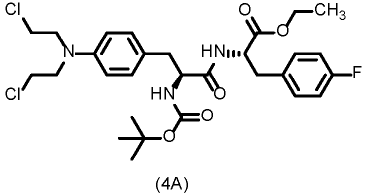

10. Le procédé selon les revendications 8 ou 9, dans lequel, à l'étape b, le composé de formule (4) ou le composé de formule (4A) est déprotégé avec du HCl dissous dans de l'éther méthylique de cyclopentyle.

11. Composé de formule, 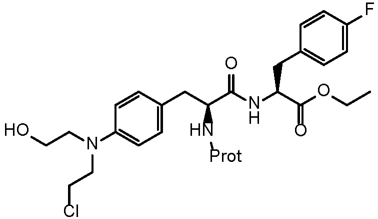
- Prot signifiant groupe protecteur d'azote.

12. Utilisation du composé selon la revendication 11, pour la préparation de melflufen, composé de formule (1) ou d'un sel en dérivant.

13. Une forme solide du composé de formule (2A), forme A, 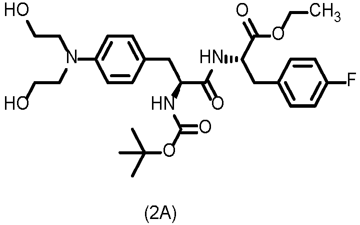 **caractérisée par** un spectre XRPD présentant les valeurs 2θ de 5,7°, 17,4° et 19,0°, 2θ (± 0,2 degrés 2θ).

14. La forme solide selon la revendication 13 **caractérisée par** un spectre XRPD présentant les valeurs 2θ de 5,7°, 8,5 ,17,4°, 19,0° et 21,0° 2θ (±0,2 degrés 2θ).

15. Une forme solide du composé de formule (6A) Forme B, **caractérisée par** un spectre XRPD présentant les valeurs 2θ de 13,0°, 14,9° et 19,2° 2θ (± 0,2 degrés 2θ)

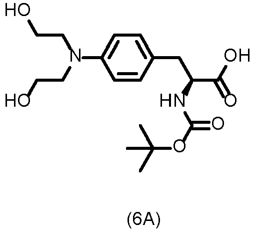

16. La forme solide selon la revendication 15, **caractérisée par** un spectre XRPD présentant les valeurs 2θ de 11,8°, 13,0°, 14,9°, 19,2° et 20,6° 2θ (± 0,2 degrés 2θ).
